(19) **Europäisches Patentamt**
**European Patent Office**
**Office européen des brevets**

(11) **EP 2 735 266 B1**

(12) **EUROPEAN PATENT SPECIFICATION**

(45) Date of publication and mention
of the grant of the patent:
**22.04.2015 Bulletin 2015/17**

(51) Int Cl.:
***A61B 5/01*** *(2006.01)*  ***A61B 5/00*** *(2006.01)*
***A61B 5/107*** *(2006.01)*

(21) Application number: **13193533.0**

(22) Date of filing: **19.11.2013**

(54) **Method and apparatus for measurement of body fat on abdominal cross section including umbilicus**

Verfahren und Vorrichtung zur Messung des Körperfettgehalts im abdominalen Querschnitt mit Umbilicus

Procédé et appareil de mesure de graisse corporelle en section transversale des abdominaux comprenant l'ombilic

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.11.2012 JP 2012255000**

(43) Date of publication of application:
**28.05.2014 Bulletin 2014/22**

(73) Proprietor: **Hutech Laboratory Co., Ltd.**
**Hokkaido 059-1303 (JP)**

(72) Inventors:
• **Shimano, Yumi**
**Hokkaido 059-1303 (JP)**
• **Shimano, Kenjiro**
**Hokkaido 059-1303 (JP)**

(74) Representative: **MacDougall, Alan John Shaw et al**
**Mathys & Squire LLP**
**The Shard**
**32 London Bridge Street**
**London SE1 9SG (GB)**

(56) References cited:
**WO-A2-2009/067501     US-A1- 2006 030 783**

• **VAN MARKEN LICHTENBELT W D ET AL: "Effect of individual characteristics on a mathematical model of human thermoregulation", JOURNAL OF THERMAL BIOLOGY, PERGAMON PRESS, OXFORD, GB, vol. 29, no. 7-8, 1 October 2004 (2004-10-01), pages 577-581, XP004586719, ISSN: 0306-4565, DOI: 10.1016/J.JTHERBIO. 2004.08.081**

**Description**

BACKGROUND OF THE INVENTION

1. Field of the Invention

The present invention relates to a method and a device for measurement of body fat of a subject, in particular for evaluation of fat on the abdominal cross section including the umbilicus.

2. Description of Related Art

Medical treatment for obese patients is prevalent. In such treatment, patients are given advice on a diet, exercise, etc. Obese patients sometimes find dietary restriction so stressful that it is important as a part of treatment to help them to maintain their motive for restricting their diet. If obese patients are able to realize how successful they have been in their attempt to lose weight by being kept informed of the effect of the treatment that they have received, they will be encouraged to carry on. Furthermore, it is obvious that, for appropriate obesity treatment based on medical knowledge, doctors need data which allows them to understand the effectiveness of the treatment.

Among methods of acquiring data about results of obesity treatment, the simplest way is measurement of weight. Measurement of weight is so easy that one can conduct it on a daily basis, and a decrease in weight might be regarded as proof of successful treatment for obesity. However, weight meter readings are extremely sensitive to only a small amount of food or drink taken before the measurement. On the other hand, development of muscle can lead to an increase in weight even when fat has been reduced. It is, therefore, impossible to judge only from weight measurement whether obesity is being eliminated. It is inappropriate to use such highly uncertain numerical information as the basis of medical treatment, and patients cannot be encouraged by looking at figures which do not accurately reflect the results of their treatment.

It is considered that the most accurate measurement of change in the amount of fat can be realized by CT (Computed Tomography) scanning. However, patients are subject to rather high radiation exposure levels by CT scanning. For this reason, frequency of CT scans should be limited: daily or weekly scans are not acceptable. Another demerit of CT scanning is its high cost.

There is a body fat meter which can evaluate an amount of body fat from bioelectric impedance (e.g. Japanese Unexamined Patent Application Publication No. 2005-152061, Japanese Unexamined Patent Application Publication No. 2011-25071 and Japanese Unexamined Patent Application Publication No. 2009-261435). When a body fat meter based on bioelectric impedance is used, electrodes contacting bodily parts such as palms and feet superimpose voltage on the body so as to measure bioelectric impedance from which the amount of fat is estimated through a prearranged algorithm. As body fat can be measured easily, this technique seems suitable for acquisition of data which indicates the effectiveness of obesity treatment.

SUMMARY OF THE INVENTION

Nonetheless, the body fat measurement technique based on bioelectrical impedance has been proved to be ineffective for obtaining a highly accurate measurement. For example, the exact position where the fat measurement was really conducted is unclear in the bioelectrical impedance approach because the exact route of the electric current cannot be examined. This means that the measured impedance changes depending on the route of the current. Even if the measured impedance is corrected, this will not result in a highly accurate evaluation of fat.

One exemplary object of the exemplary embodiments is to provide a method and device capable of measuring body fat accurately in a simple manner and at a low cost.

A method of fat measurement according to an aspect of the present invention includes:

a measuring step measuring a distribution of skin surface temperature of the subject;
a first operation step evaluating temperature gradient on the skin surface based on the measured skin temperature;
a second operation step calculating a skin temperature distribution by conducting a numerical heat conduction analysis on a prearranged sample body composition with the temperature gradient imposed as the boundary condition;
a comparing step comparing the skin temperature distributions obtained in the measuring and second operation steps;
a modification step modifying the body composition of the sample in a case where the two skin temperature distributions in the comparing step disagree; and
repeating the second operation step and the modification step to make the two skin temperature distributions sufficiently close.

An apparatus for body fat measurement according to an aspect of the present invention includes:

a measuring means that measures a distribution of skin surface temperature of a subject;
a first operation means that evaluates temperature gradient on the skin surface based on the measured skin temperature;
a second operation means that calculates a skin temperature distribution by conducting a numerical heat conduction analysis on a prearranged sample body composition with the temperature gradient imposed as the boundary condition;
a comparing means that compares the measured and calculated skin temperature distributions; and
a modifying means that modifies the body composition of the sample in a case where a difference between the two skin temperature distributions is detected by the comparing means;
wherein, until the calculated skin temperature distribution is sufficiently close to the measured skin temperature distribution, the modification to the sample body composition by the modification means and the calculation of the skin surface temperature distribution by the second operation means are repeated.

According to the present invention, it is possible to provide a method and device capable of measuring body fat accurately in a simple manner and at a low cost.

The above and other objects, features and advantages of the present invention will become more fully understood from the detailed description given hereinbelow and the accompanying drawings which are given by way of illustration only, and thus are not to be considered as limiting the present invention.

BRIEF DESCRIPTION OF THE DRAWINGS

Fig.1A shows a simplified model outlining a body composition on the abdominal cross section including the umbilicus;
Fig.1B shows a simplified model outlining a body composition on the abdominal cross section including the umbilicus;
Fig.2A shows the temperature distribution calculated for the corresponding model;
Fig.2B shows the temperature distribution calculated for the corresponding model;
Fig.3A shows a body composition model made from a CT scan of the abdominal cross section including the umbilicus;
Fig.3B shows a body composition model made from a CT scan of the abdominal cross section including the umbilicus;
Fig.4A shows a result of temperature measurement on the abdominal skin surface by thermography;
Fig.4B shows a result of temperature measurement on the abdominal skin surface by thermography;
Fig.5 is a graph which shows distributions of a measured skin temperature along the left halves of the abdomens from FRONT to BACK;
Fig.6A shows temperature and heat flux vector distributions calculated from two-dimensional steady-state heat conduction equation for steady states (4);
Fig.6B shows temperature and heat flux vector distributions calculated from a two-dimensional steady-state heat conduction equation for steady states (4);
Fig.7 is a diagram showing a flow of process operations in a first exemplary embodiment 1;
Fig.8 shows an example of computational domains partitioned into numerous subdomains, which are called computational cells or control volumes, in application of the finite volume method;
Fig.9 shows decomposition of a boundary curve into line-segments in the boundary element method;
Fig.10 is a diagram summarizing inputted and output information in the case when the boundary element method is applied to the present invention;
Fig.11 is a diagram summarizing inputted and output information in the case when a temperature gradient is given on the skin surface;
Fig.12 is a diagram summarizing inputted and output information in the case when an inverse problem analysis is carried out with the boundary element method;
Fig.13 is a diagram showing the flow of process operations in a second exemplary embodiment; and
Fig. 14 is a diagram summarizing inputted and output information in the analysis for constructing a database relating to Variation 1.

DESCRIPTION OF THE EXEMPLARY EMBODIMENTS

Exemplary embodiments according to the present invention are explained hereinafter with reference to the drawings.

The inventors focused on the fact that skin surface temperature varies depending on the size of the body and considered that the difference in skin temperature should correlate with the difference in the amount of body fat. Then, they conceived the possibility of conducting fat measurement based on data of skin surface temperature. If body fat could be evaluated from skin surface temperature, this approach would be extremely useful because skin temperature can be measured very easily. After making a diligent effort in their research, the inventors came to the conclusion that the amount of body fat can be evaluated from skin surface temperature through heat conduction analysis.

First of all, the basic principle of the present invention that body fat can be evaluated by a heat conduction analysis will be explained below.

(Basic principle)

The basic principle of the approach for measurement of body fat on the abdominal cross section including the umbilicus based on a heat conduction analysis will now be explained.

The macroscopic thermal environment of the human body is mainly determined by (A1) heat generated by metabolism and (A2) heat transferred from the skin surface to the surroundings. Here, heat lost through respiration is not considered because such heat is much less than A2 and because the abdominal cross section in question is located far from the respiratory tract. No considerable temporal variation in body temperature occurs because (A1) heat generation due to metabolism balances (A2) cooling on the skin surface. In other words, although the human body receives a certain amount of heat per unit time due to (A1) heat generation by metabolism, no temporal change in temperature in any part of the body can occur because same amount of heat is removed from the body through the skin surface (the cooling effect of A2).

Heat transfer in a living body will now be considered.

As heat is transferred from hot spots to cold spots, heat is always transferred from the body core with a higher temperature to the skin with a lower temperature. There are two forms of heat transfer in the body: heat conduction and convective heat transfer due to blood flow. As far as the abdominal cross section including the umbilicus is concerned, heat conduction is dominant because, in the abdomen, there are no large blood vessels through which heat can be transported to the skin surface.

A spatial difference in temperature causes heat transfer. When heat is transferred by heat conduction, the amount of heat transferred is proportional to the corresponding temperature gradient. Let $x$ be the direction in which heat is conducted. Heat flux $q$ [W/m$^2$] representing heat transferred per unit time through unit area is expressed by the following equation (1).

$$q = -\lambda \frac{dT}{dx} \quad \cdots\cdots \quad (1)$$

Here, $\lambda$, the constant of proportionality, stands for thermal conductivity with the dimension of W/(m $\cdot$ K). It is likely that less heat is transferred through a material with a lower thermal conductivity. In human bodies thermal conductivity of muscle and skin is approximately 0.4W/(m$\cdot$ K) while thermal conductivity of body fat is only half of that, i.e. approximately 0.2W/(m$\cdot$ K). Objects which hinder heat conduction like fat are called thermal resistances.

When a thermal resistance like fat is located on the path of heat conduction, temperature gradient dT/dx should increase so as to maintain the same amount of heat flux. An increase in temperature gradient results in the more detectable changes mentioned below.

(B1) The hot section becomes hotter (what is called a state of heat accumulation).

(B2) The cold section becomes colder.

When human bodies are in a state of rest, the body core temperature is maintained in the range between 37 and 38 degrees Celsius regardless of the amount of body fat. Therefore, (B2) shown above mainly occurs in bodies with more fat and, as a result skin temperature is lowered because skin is the coldest section in each body.

In addition, when a body gains fat, not only the abdomen girth but also the distance between the body core and the skin surface become longer. To compensate for this, a further drop in the skin temperature occurs. This is because a longer distance between the body core and skin surface brings about a decrease in temperature gradient unless the temperature difference between the hot and cold sections is expanded. Thus, it can be concluded that there is a correlation between the amount of fat and the skin temperature, which enables a fat measurement based on the skin temperature to be conducted.

The important features in the present invention are summarized as follows:

(C1) A constant amount of heat is always transferred from the body core to the skin surface.
(C2) Body fat acts as a heat resistance.
(C3) The body core temperature is kept almost constant.

(Heat conduction analysis: a prerequisite for the method of fat evaluation)

Here, an explanation of heat conduction analysis, which is a prerequisite for the method of fat evaluation, is provided.

The influences of the heat resistance were explained in the above discussion, with the one-dimensional heat conduction in the x-direction being assumed. In actual human bodies, heat can be transferred three-dimensionally in the $x$, $y$ and $z$-directions, and distributions of temperature and heat flux are more complicated than in the one-dimensional heat conduction. Paths of heat from the body core to the skin surface vary, depending on the distribution of body fat. However, a difference in the skin temperature still reflects a difference in the amount of body fat even when multi-dimensional heat conduction occurs.

Therefore, in the present invention, internal body temperature and heat flux vector distributions are obtained by a numerical solution of the following steady-state heat conduction equation.

$$\frac{\partial q_x}{\partial x} + \frac{\partial q_y}{\partial y} + \frac{\partial q_z}{\partial z} = -\lambda\left(\frac{\partial^2 T}{\partial x^2} + \frac{\partial^2 T}{\partial y^2} + \frac{\partial^2 T}{\partial z^2}\right) = q_{in} \quad \cdots\cdots \quad (2)$$

wherein ,

$$q_x = -\lambda\frac{\partial T}{\partial x} \quad , q_y = -\lambda\frac{\partial T}{\partial y} \quad , q_z = -\lambda\frac{\partial T}{\partial z} \quad \cdots\cdots \quad (3)$$

Here, $q_{in}$ is a quantity with the dimension of W/m$^3$, which represents internal heat generation per unit time in unit volume. This corresponds to heat generation by metabolism in human bodies. The coordinate axes are defined so that the $x$-$y$ plane is identical to the cross section of the abdomen including the umbilicus. The $z$-axis perpendicular to the $x$-$y$ plane is almost parallel to the spinal cord.

For evaluation of body fat located on the abdominal cross section including the umbilicus, only two-dimensional distributions of temperature and heat flux on the $x$-$y$ plane are necessary. The second order derivative in the $z$-direction in Equation (2) is negligible because, on the abdominal cross section, the body composition is not expected to change much in the $z$-direction. In virtue of this arrangement, Equation (2) can be replaced by the two-dimensional steady-state heat conduction equation written below, which can be solved at a much lower computational cost.

$$-\lambda\left(\frac{\partial^2 T}{\partial x^2} + \frac{\partial^2 T}{\partial y^2}\right) = q_{in} \quad \cdots\cdots \quad (4)$$

Note that it is necessary to include in $q_{in}$ the effect of heat brought to the abdominal cross section by blood flow in the z-direction.

(How to determine $q_{in}$)

For solution of Equation (4), $q_{in}$ should be given beforehand.

How to determine $q_{in}$ will now be explained.

As explained above, $q_{in}$ on the right hand side of the two dimensional heat conduction equation (4) is a quantity with the dimension of W/m$^3$ ,which means the amount of heat given to a material of unit volume per unit time.

To calculate a temperature distribution across the cross section of the abdomen including the umbilicus, the following effects should be taken into consideration:

(D1) the effect of metabolism in muscle and intestines;

(D2) the effect of heat brought by blood flow through vessels which penetrate the abdominal cross section.

The sum of the two effects is given as $q_{in}$.

((D1) heat generation in muscle and intestines due to metabolism)

The Harris-Benedict formula is used to determine a total basal metabolic rate. The Harris-Benedict formula is given in Reference 1 (J.A. Harris and F.G. Benedict: A Biometric Study of Basal Metabolism in Man, The Carnegie Institution of Washington, (1919)).

This is a formula widely used for evaluation of basal metabolic rates of healthy persons in a state of rest. According to the formula, the basal metabolic rate [kcal/day] of the subject is calculated through a linear function of weight, height and age. Then, $q_{in}$ is evaluated based on the assumption that 38% of the total metabolic heat is attributed to muscle and 7 % to intestines, respectively. These percentages are given in Reference 2 (S. Yokoyama: Heat Transfer Phenomena in Living bodies, Hokkaido University Press, (1993)).

((D2) heat brought by blood flow through vessels which penetrate the abdominal cross section)

Large blood vessels penetrating the cross section of the abdomen including the umbilicus are the aorta, inferior vena cava, superior mesenteric artery, superior mesenteric vein, inferior mesenteric artery and inferior mesenteric vein. Let $q$ be heat flux transferred from blood passing through the large vessels to the surrounding tissue, and $q$ is expressed by the following formula.

$$q = h\Delta T \quad \cdots\cdots \quad (5)$$

Here, $\Delta T[K]$ represents a temperature difference between blood and the surrounding tissue while $h$ [W/(m$^2$ • K)] stands for a heat transfer coefficient. Assume that the blood vessel is a circular pipe with a diameter of $D$ and a length of $L$, and the heating surface area is equal to $\pi DL$. The amount of heat transferred per unit time is, then, expressed as $\pi DLq$ [W] as the product of the heating surface area $\pi DL$ and the heat flux $q$. By dividing this amount of heat by the volume of the vessel $\pi D^2 L/4$, one can obtain a quantity equivalent to $q_{in}$ [W/m$^3$].

The heat transfer coefficient $h$ for a fully developed flow can be calculated by Hausen's formula written as follows.

$$h = \left[ 3.66 + \frac{0.0668 \cdot \mathrm{Re} \cdot \mathrm{Pr}\ (D/L)}{1 + 0.04 \left[ \mathrm{Re} \cdot \mathrm{Pr}\ (D/L) \right]^{2/3}} \right] \cdot \frac{\lambda_a}{D}$$

*wherein,*

$$\mathrm{Re} = \frac{\rho u D}{\mu} \quad , \mathrm{Pr} = \frac{c\mu}{\lambda_a} \quad\quad \cdots\cdots (6)$$

Here, $\lambda_a$ [W/(m • K)] is thermal conductivity of blood.

Re and Pr stand for the Reynolds number and the Prandtl number, respectively. Parameters $u$ [m/s], $\mu$ [Pa • s] and $c$ [J/(kg • K)] in the formulae of Re and Pr represents mean blood velocity, blood viscosity and specific heat of blood, respectively.

The mean velocity $u$ in the formula of the Reynolds number can be calculated from mean volumetric flow rate $Q$ [m$^3$/s] in the following manner.

$$u = \frac{4Q}{\pi D^2} \quad \cdots\cdots \quad (7)$$

It, therefore, follows that $h$ in Equation (6) can be calculated if the mean volumetric flow rate in each vessel is available. $Q$ can be determined according to the well-known Murray's law, which tells us that the volumetric flow rate is proportional to the cube of the vessel diameter. Murray's law is given in Reference 3 (C.D. Murray: The Physiological Principle of Minimum Work: I. The Vascular System and the Cost of Blood Volume, Proceedings of the National Academy of Sciences of the United States of America, (1926), Vol.12, No.3, pp 207-214).

(Validation with simplified models)

It is demonstrated by a calculation with simplified models that the approach based on heat conduction analysis explained above is valid for conducting fat measurement.

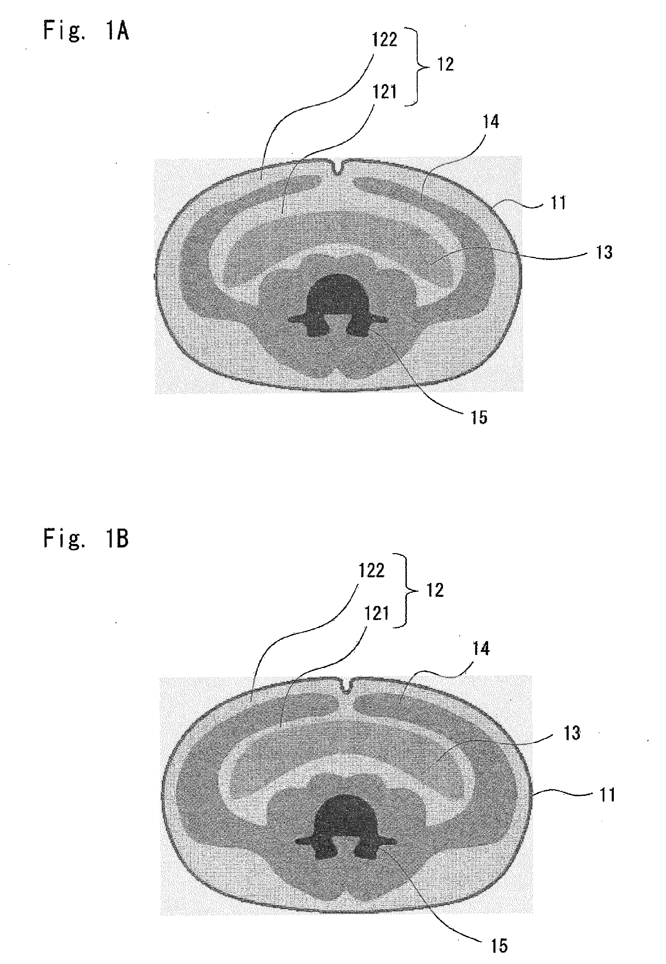

Figs.1A and 1B show simplified body composition models of abdominal cross sections including umbilici.

The model in Fig.1A is called Model A while the one in Fig.1B is called Model B.

Both models have an abdominal girth of 810 mm, and the whole area of the cross section is the same in both models.

Here, the reference numerals used in Figs.1A and 1B will be briefly explained. "12" indicates fat, "13" indicates intestines and "14" indicates an abdominal muscle, all of which are surrounded by skin indicated by "11". Bone (vertebra) indicated by 15 is located at the center of this model.

The amount of fat 12 is 209.0cm$^2$ in Model A and 170.2 cm$^2$ in Model B: a larger amount of the fat 12 is present in Model A. This difference between the respective amounts of fat in the two models is solely attributed to the difference in the amount of visceral fat 121 located between the abdominal muscle 14 and intestines 13. The amount of subcutaneous fat 122 present in Model A is the same as that in Model B.

As Model A contains more fat than Model B, less muscle 14 is arranged in Model A than in Model B so that the total area of Model A is the same as that of Model B. More precisely, there is 167.0 cm$^2$ of the muscle 14 in Model A in contrast to 204.6 cm$^2$ in Model B, which means that Model A has approximately 20% less muscle than Model B.

Although the body composition and structures considered here do not always agree with those in real human bodies, they are sufficient for a basic examination to validate the method in the present invention.

Temperature distributions in the two models were calculated by applying the finite volume method with the unstructured grid system for solution of the two-dimensional heat conduction equation (4). Constant heat $q_{in}$=780W/m$^3$ was given to abdominal muscle and intestines. The computational domain in each model was partitioned into approximately 40000 triangular computational cells. It was assumed that the temperature along the skin surface was constant and that the average temperature of the intestines was 37 degrees Celsius. These two conditions were imposed as constraints.

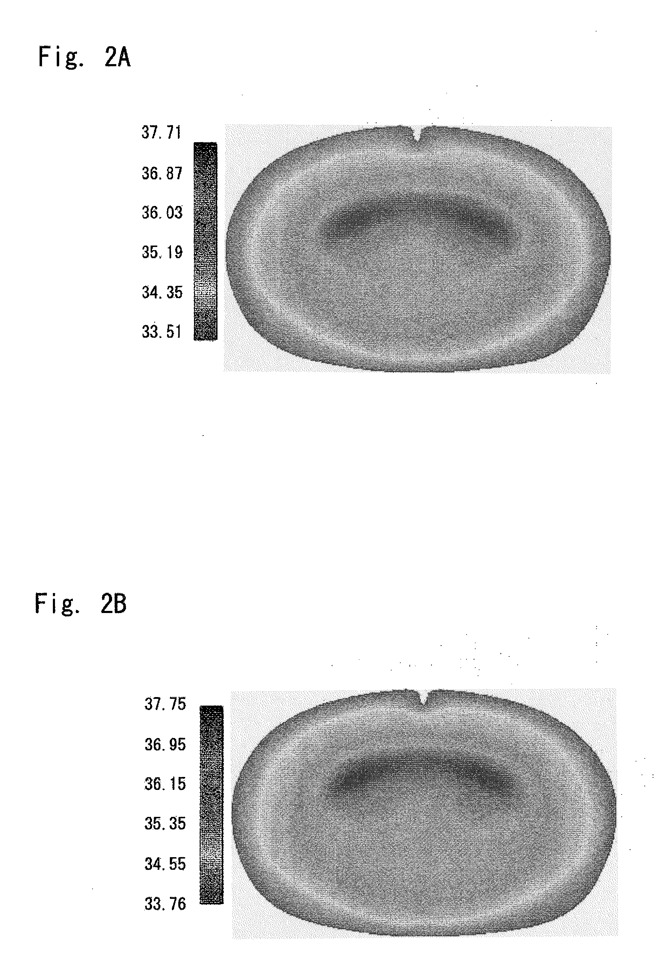

The obtained computational results are depicted in Figs.2A and 2B.

Fig.2A shows the temperature distribution in Model A and Fig.2B shows that in Model B. In both models, the lowest temperature was recorded in the skin 11. The values of the lowest temperature were 33.51 degrees Celsius in Model A and 33.76 degrees Celsius in Model B. The resolution of modem temperature sensors is high enough to detect the difference between the lowest temperatures in the two models.

It will be recalled that Model A had approximately 20% less muscle than Model B.

As constant heat $q_{in}$ = 780 W/m$^3$ was given to abdominal muscle and intestines in both models, it follows that the heat generated in Model A was 20% less than that generated in Model B.

If thermal conductivity had been constant over the computational domain regardless of the body composition such as the amounts of the muscle 14 and fat 12, a temperature gradient induced in Model B would have been 20% steeper than that induced in Model A and the skin temperature in Model B would have been lower than in Model A.

(As the same average temperature of the intestines, 37 degrees Celsius, was imposed in both models, Model B with more internal heat generation than that of Model A would have required a lower temperature in the skin 11 than that in Model A for more cooling than that in Model A.)

Nonetheless, a skin temperature lower than that in Model B was actually calculated in Model A. It can be asserted that the lower thermal conductivity in fat 12 accounts for the lower skin temperature in Model A.

Two human bodies having different amounts of fat but the same abdominal girth are rarely seen; a body with more fat than another body is likely to have a longer abdominal girth than that of the other body, which means the former body has a longer heat transmission distance than that of the latter body. Therefore, in actual obese human bodies, a reduction in skin temperature greater than that in simplified Model A is considered to occur. It can be concluded that the amount of body fat can be evaluated from the skin temperature because the sensitivity of the latter to the former is sufficiently large.

(Validation with human body models)

Examples of a heat conduction analysis applied to real human bodies are shown here. CT scans of two volunteers were used to make up models.

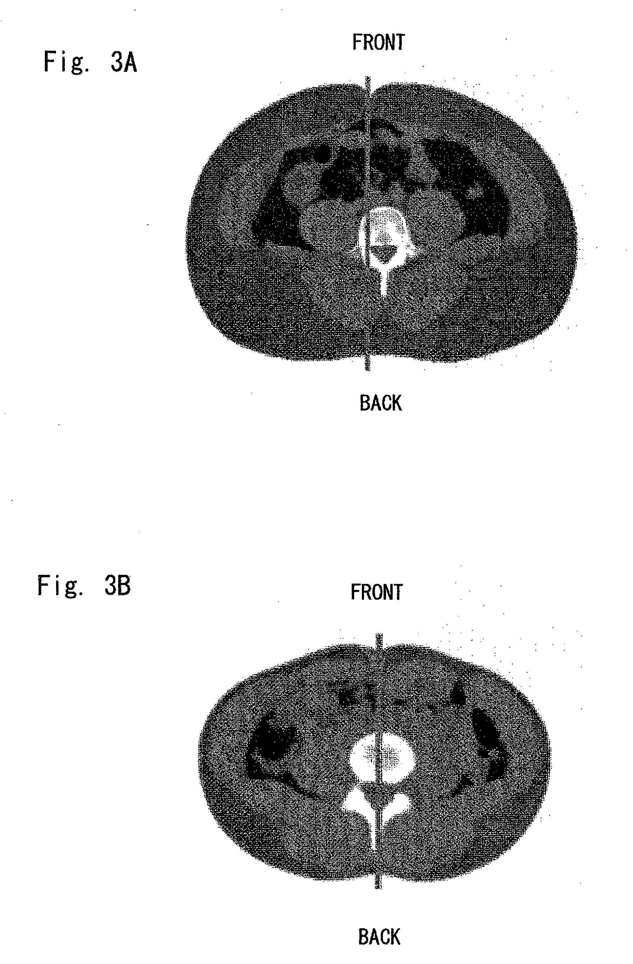

Real Model A and Real Model B respectively shown in Fig.3A and Fig.3B were made from CT images of the two volunteers.

Fat area estimated from the CT images is 398.1 cm$^2$ in Real Model A and 56.5 cm$^2$ in Real Model B.

Distributions of the skin temperature of the two volunteers were also measured by thermography.

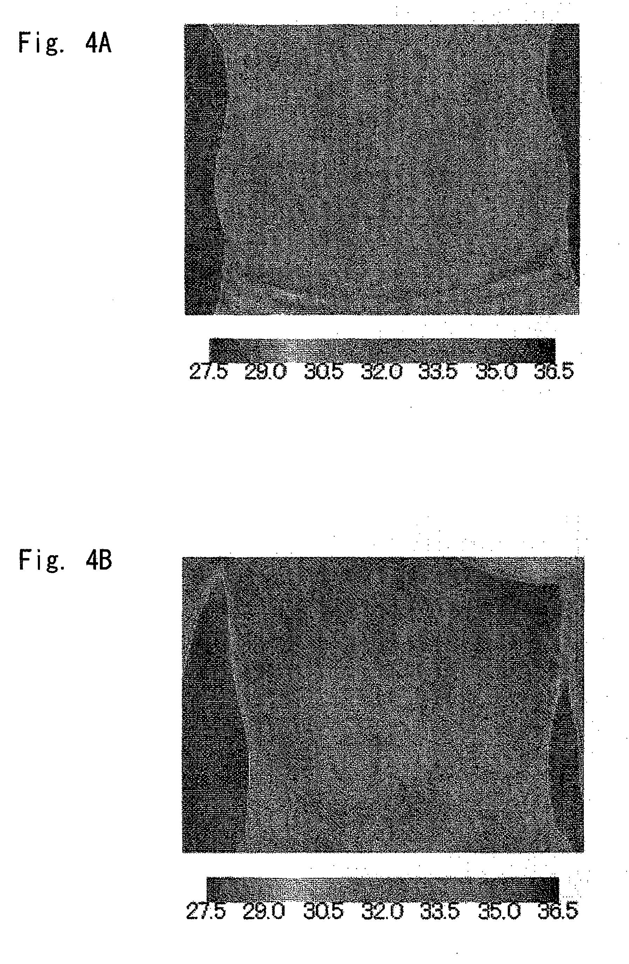

Figs.4A and 4B show results of the temperature measurement by thermography.

Fig.4A and Fig.4B correspond to Fig.3A and Fig.3B, respectively. The distributions of the skin temperature obtained in the temperature measurement were imposed as the boundary conditions for solution of the two-dimensional steady-state heat conduction equation (4).

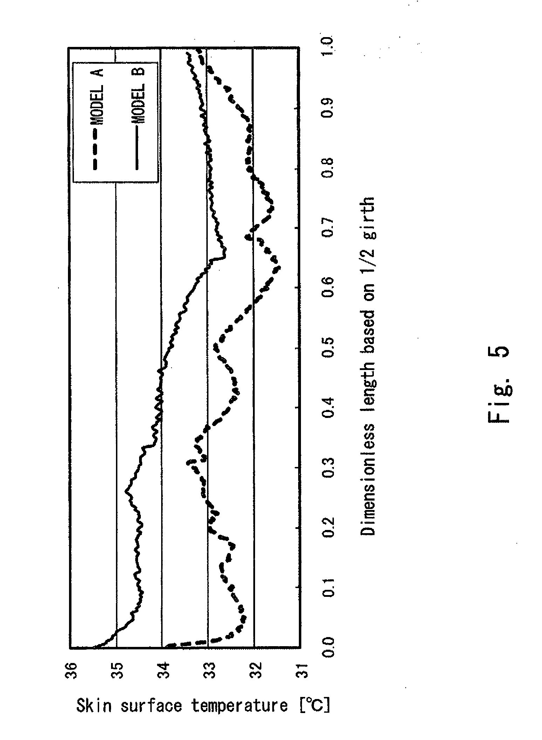

Here, imaginary vertical lines passing through the umbilici are considered. In each figure, the intersectional point with the skin surface at the umbilicus is defined as FRONT and that in the posterior part is defined as BACK. Fig.5 shows distributions of the measured skin temperature along the left halves of the abdomens from FRONT to BACK. It can be clearly seen in Figs.4 and 5 that the skin surface temperature in Real Model A, which had more body fat than Real Model B, was lower than that of Real Model B.

Internal temperature distributions were obtained by solving the two-dimensional steady-state heat conduction equation (4) and imposing the skin temperature distributions measured by thermography as the boundary conditions. Internal heat flux distributions were also obtained from the temperature distributions.

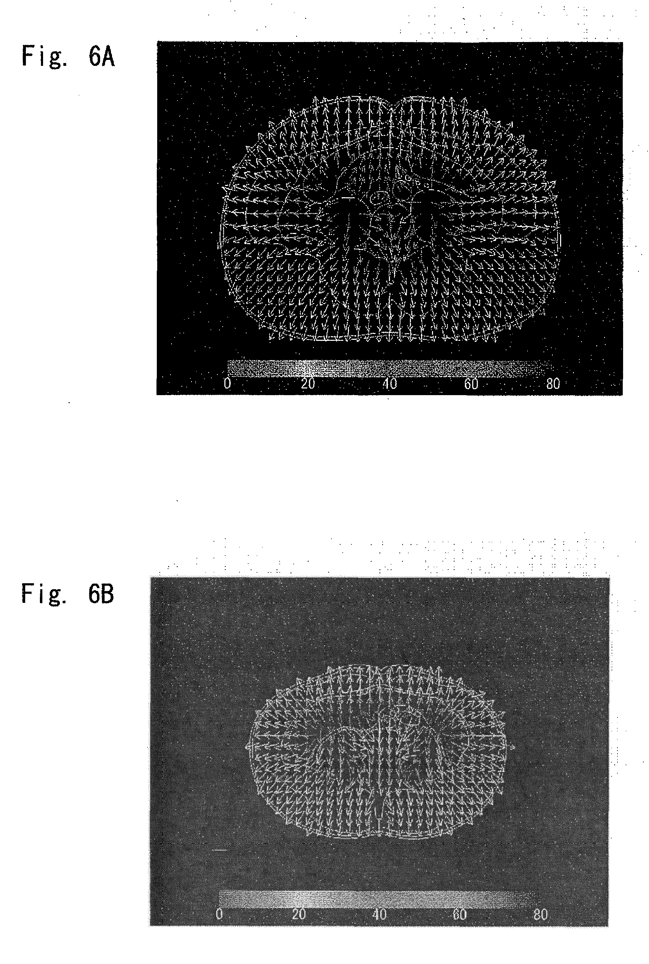

The results are shown in Figs.6A and 6B.

In Real Model A, heat generated in the intestines and the blood vessels located near the intestines moved forward in the direction of the ventral surface.

Heat moving toward the dorsal surface in Real Model A was mainly generated in the muscle surrounding the vertebra. In sharp contrast to Real Model A, in Real Model B, heat generated in the intestines and the blood vessels located near the intestines was transferred in the ventral and dorsal surfaces at the same time. The above results demonstrate that temperature and heat flux distributions across the cross sections of the abdomens differed distinctively between the two subjects with different amounts of body fat.

(First exemplary embodiment)

A first exemplary embodiment of the present invention will be explained.

It will be explained how to apply practically the method of evaluating body fat based on heat conduction analysis discussed above to actual obese patients.

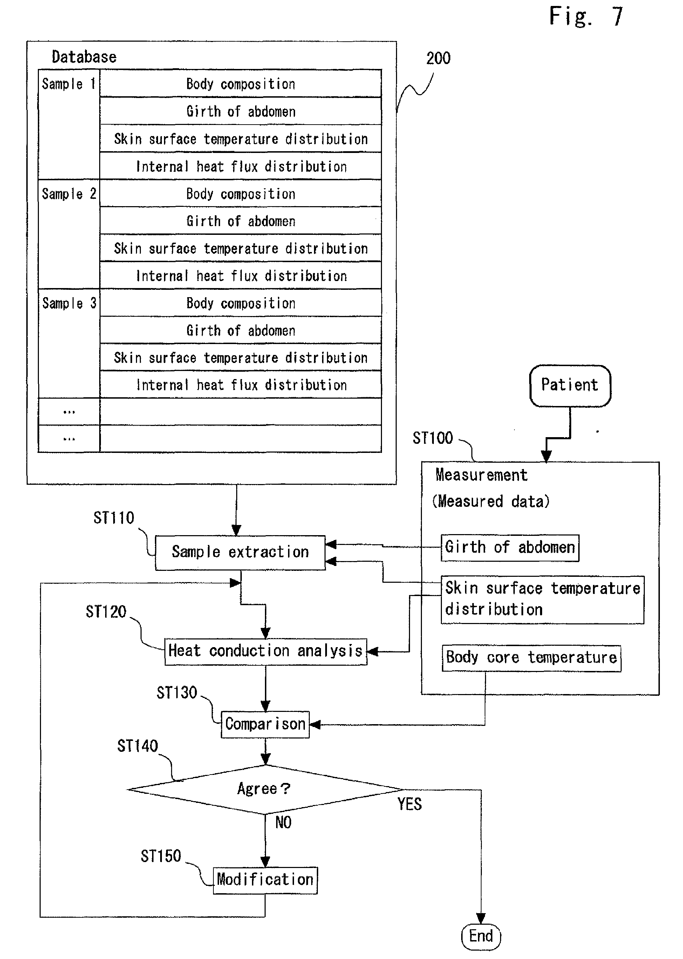

Fig.7 is a diagram showing the flow of process operations in the first exemplary embodiment.

First of all, it is necessary to construct a database 200 in advance for this exemplary embodiment. Multiple sets of sample data are stored in the database 200. For construction of the database 200, acquisition of basic data, which will be used for reference, is carried out with as many human samples as possible. A set of data related to each sample consists of body composition, girth of abdomen, distributions of skin surface temperature and internal heat flux.

Data about the body composition is obtained from a corresponding CT scan while the distribution of the skin temperature can be measured by thermography. The distribution of internal heat flux is calculated by the aforementioned approach in which the two-dimensional steady-state heat conduction equation (4) is solved on the body composition provided by CT scanning with a measured skin temperature imposed as the boundary condition. If such a database with multiple sets of basic data is prepared for reference, an unknown body composition of a new subject can be, in turn, estimated from his/ her skin temperature distribution.

A subject's own data, which was measured in the past, can also be accepted as an entry in the database. For example, when a new patient is admitted in hospital, a set of the basic data is measured and stored in the database. Then, in a routine check-up during the period of the new patient's stay in hospital, his/her own data taken in the past can be utilized in order to examine his/her present state with the method of the invention, so that frequent CT scans can be avoided. His/her own past data stored in the data is considered to be useful in estimating his/her present state.

Measurements are made of the abdomen girth, skin surface temperature and body core temperature of a patient whose body fat is going to be evaluated by the present method (ST100). Skin surface temperature can be measured by thermography. A measured rectum temperature can be used as the body core temperature. Otherwise, the body core temperature can be evaluated from an axillary or sublingual temperature through a prearranged correction formula. Subsequently, the sample closest to the patient in terms of abdomen girth and skin surface temperature is extracted from samples in the database (ST110). One possible approach in this process is to shortlist several promising samples based on only the abdomen girth data, and then to select one sample from the shortlisted samples, referring to the averaged skin temperature. Furthermore, there is another possible approach in which shapes of skin temperature curves such as those shown in Fig.5 are examined: samples with skin temperature curves similar in shape to the patient's curve are chosen. For example, in one of the temperature curves drawn in Fig.5, a temperature is lower where a dimensionless length is in the range from 0.5 to 0.8, and this tendency is considered to reflect the amount of body fat. Good samples similar to the patient in regards to the quantity of body fat can be efficiently extracted if ones with similar tendencies in skin temperature curves are chosen.

In this process of sample extraction, automatic extraction is usable where a program embodying a prearranged algorithm is installed and executed on a computer.

Subsequently, a heat conduction analysis is carried out on the body composition of the extracted sample (ST120). Note that the skin temperature distribution of the patient is imposed as the boundary condition; not that of the sample.

Subsequently, the body core temperature calculated by heat conduction analysis (ST120) is compared with that measured in advance (ST130). The body composition in the selected sample is not necessarily identical to that of the patient, and this difference in body composition is reflected in the difference between the measured and calculated core temperatures. In other words, if the body composition of the sample is different from that of the patient (ST140: NO), the body core temperature calculated by heat conduction analysis deviates from the real body core temperature. In order to obtain the correct body composition of the patient, the body composition of the sample is modified so that the difference in body core temperature is minimized (ST150).

In a modification to the body composition of the sample (ST150), the amounts of fat and muscle are changed mainly. A modification to vertebra or intestines is not always required because there is rarely significant variation in sizes of vertebra and intestines among different individuals, and because the selected sample is similar to the patient in physique (to be sure, vertebra or intestines can be modified if necessary).

When the amounts of fat and muscle are changed, one possible approach in this process is homothetic transformation in which an object size varies while its shape remains the same, because a dramatic change in the position and shape of the fat and muscle is unlikely. The degree and location of the modification are determined by looking at the heat flux distribution obtained by a heat conduction analysis.

In this modification process, it is preferable that a program embodying a prearranged algorithm can be installed and executed on a computer. However, it is also possible for an operator to carry out successive modifications manually based on insights into heat conduction and a doctor to carry out the same based on insights into medicine.

The processes explained above are reiterated until the body core temperature calculated by the heat conduction analysis agrees with the measured body core temperature of the patient. When the calculated and measured body core temperatures agree (ST140: YES), the body composition used in the heat conduction analysis is considered to be the same as that of the patient. Therefore, the proportion and amount of fat in the calculated body composition are those of the patient. In this manner, the amount of body fat in the patient's abdomen can be evaluated by heat conduction analysis.

In this embodiment, the following merits are expected.

There were only two methods available for evaluation of fat in human bodies, CT scanning and fat measurement based on bioelectric impedance.

However, CT scanning is not only expensive but also invasive due to radiation exposure, while highly accurate measurement is impossible with the impedance method.

In contrast to the conventional methods, only abdomen girth, skin surface temperature and body core temperature need to be measured, only a low cost is incurred and no invasive operations are included in the exemplary embodiment. It is also noteworthy that the amount of fat can be evaluated fairly accurately by heat conduction analysis based on a relationship between skin temperature and body fat. Thus, frequent measurements can be conducted and, for example, as a part of obesity treatment, a patient can be provided with information about any recent change in the amount of his/her body fat.

The first exemplary embodiment also enables doctors engaged in obesity treatment to make more proper judgments and give more adequate advice to patients.

(Second exemplary embodiment)

A second exemplary embodiment derived by improvement of the first exemplary embodiment will now be explained.

In the first exemplary embodiment, the finite volume method was used for solution of the two-dimensional steady-state heat conduction equation (8) on the cross section of the abdomen including the umbilicus.

$$-\lambda \left( \frac{\partial^2 T}{\partial x^2} + \frac{\partial^2 T}{\partial y^2} \right) = q_{in} \quad \cdots\cdots \quad (8)$$

In the second exemplary embodiment, the boundary element method replaces the finite volume method.

The reason for this is as follows.

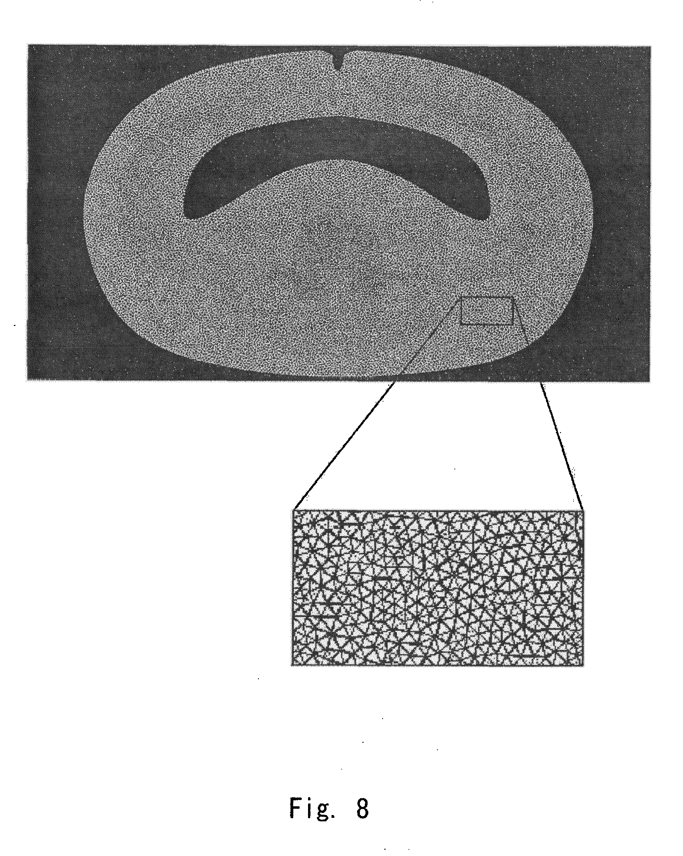

As shown in Fig.8, a computational domain is partitioned into numerous subdomains, which are called computational cells or control volumes in the finite volume method. This process is called grid generation. Although computational cells can be in any arbitrary shape, triangular or rectangular shapes are practical and convenient choices in a two-dimensional calculation.

It is, however, widely known that grid generation takes a long time even when only simple rectangular cells are used. For example, it takes approximately 20 minutes to generate approximately 40000 triangular cells on an abdominal cross section. Recall that heat conduction analysis is repeatedly conducted while modifying the body composition, when an amount of body fat is evaluated. It follows that 20 minutes is consumed for grid generation every time the body composition is modified. Even if a modification is repeated only several times, more than an hour will be inconveniently required for grid generation.

There is also a disadvantage in the finite volume method in terms of the numerical technique.

In the present method, the most probable body composition is obtained by repetition of heat conduction analysis on a trial-and-error basis until a given temperature distribution is obtained. However, no explicit relationships between skin temperature and coordinate values related to the body composition are provided as long as the finite volume method is used. Therefore, the operator is considered to undergo considerable difficulty during conducting of trial and error.

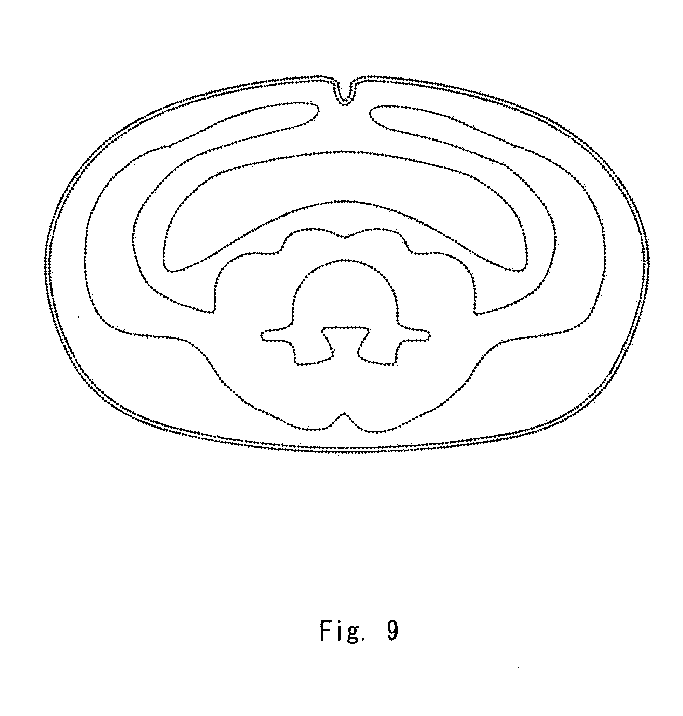

In contrast to the finite volume method, only boundary curves should be divided into line-segments and computational domains do not have to be filled with triangular or rectangular cells in the boundary element method. Fig.9 shows decomposition of a boundary curve into line-segments in the boundary element method.

In Fig.9, there are curves along which different portions meet each other. Such curves are called "internal boundaries". As the heat conduction equation is solved numerically, each internal boundary is represented by a set of discrete points. On the other hand, the curve representing the skin surface on the abdominal cross section is called an external boundary, to distinguish it from internal boundaries. The skin surface contacts no other portions but the air. A set of internal and external boundaries is simply called boundaries.

When the boundary element method is employed, the time consuming grid generation explained above is unnecessary. From the viewpoint of the computational technique, it is also advantageous for calculation of the internal body composition that, in the boundary element method, direct relationships between skin temperature and coordinate value of points composing internal boundaries are explicitly given (details will be discussed later).

(Outline of the boundary element method)

The boundary element method will now be explained.

Although boundaries are curves in view of their nature, they are usually expressed as polylines which are series of connected line-segments.

Individual line-segments composing boundaries are called boundary elements or simply elements. The *i*-th boundary element is defined as the line-segment with two end nodes $(x_i, y_i)$ and $(x_{i+1}, y_{i+1})$.

At each element, temperature $T$ and temperature gradient in the normal direction $\partial T$ are defined.

When thermal conductivity $\lambda$ and heat generation $q_{in}$ are constant, the heat conduction equation becomes Poisson's equation as follows.

$$\frac{\partial^2 T}{\partial x^2} + \frac{\partial^2 T}{\partial y^2} = C \qquad \left( C = -\frac{q_{in}}{\lambda} = const \right) \quad \cdots\cdots \quad (9)$$

When the boundary is divided into $N$ boundary elements, resultant algebraic equations in the following form are obtained by applying the boundary element method to the governing equation (9).

$$T_i = \sum_{J=1}^{N} a_{ij} T_j + \sum_{J=1}^{N} b_{ij} \partial T_j + \sum_{J=1}^{N} c_{ij} + d \qquad (i = 1 \sim N) \quad \cdots\cdots \quad (10)$$

Subscripts $i$ and $j$ given to $T$ and $\partial T$ represent the IDs of the boundary elements at which the corresponding temperature and temperature gradient are defined, and parameters $a_{ij}$, $b_{ij}$, $c_{ij}$ and $d$ are constants. Equation (10) is a linear algebraic equation including temperatures and temperature gradients defined at boundary elements.

As equation (10) holds true for every boundary element ($i = 1, ..., N$), a system of $N$ algebraic equations is obtained. As there are $N$ temperatures and $N$ temperature gradients in total, either of the two quantities must be given at each boundary element as the boundary condition so that the system of equations can be closed.

For example, if temperature gradients are given at all boundary elements, the following equation (11) is obtained by moving unknowns in Equation (10) onto the left hand side.

$$T_i - \sum_{J=1}^{N} a_{ij} T_j = \sum_{J=1}^{N} b_{ij} \partial T_j + \sum_{J=1}^{N} c_{ij} + d \qquad (i = 1 \sim N) \qquad \cdots\cdots \quad (11)$$

When Equation (11) is expressed in the matrix form, Equation (12) is obtained.

$$\mathbf{AT} = \mathbf{f} \cdots\cdots \quad (12)$$

Here, **A** is a square matrix with a size of $N$ times $N$, and **f** is a $N$-dimensional vector of which elements are given by the right hand side of Equation (11).

The details of the boundary element method explained above are about the case where there is only a single region (portion).

In the case where there are multiple portions in the computational domain like an abdominal cross section with an umbilicus, the system of equations can be closed if temperature $T$ or temperature gradient $\partial T$ is given at each external boundary element as the boundary condition.

It follows that temperature gradients at all external boundary elements constituting the skin surface can be calculated from Equation (10) if skin temperatures are given. The opposite is also true: skin temperatures can be calculated if temperature gradients are given at all external boundary elements.

There are various organs and tissues such as muscle, fat, intestines, skin and vertebra on the abdominal cross section with the umbilicus. It is stated here afresh that these are all called "portions".

(Input and output in the heat conduction analysis using the boundary element method)

Here, heat conduction analysis on the abdominal cross section by the boundary element method will be considered from the viewpoint of flow of information. Even if skin temperatures are given to all external boundary elements, temperature gradient $\partial T$ cannot be calculated because more information is still necessary to build Equation (10).

Firstly, $C$ on the right hand side of Equation (9) is necessary. Secondly, coordinate values of points composing boundaries, $(x_i, y_i)$, are required as geometrical conditions because constant terms $d$, $c_{ij}$ and coefficients $a_{ij}$, $b_{ij}$ are calculated from $(x_i, y_i)$.

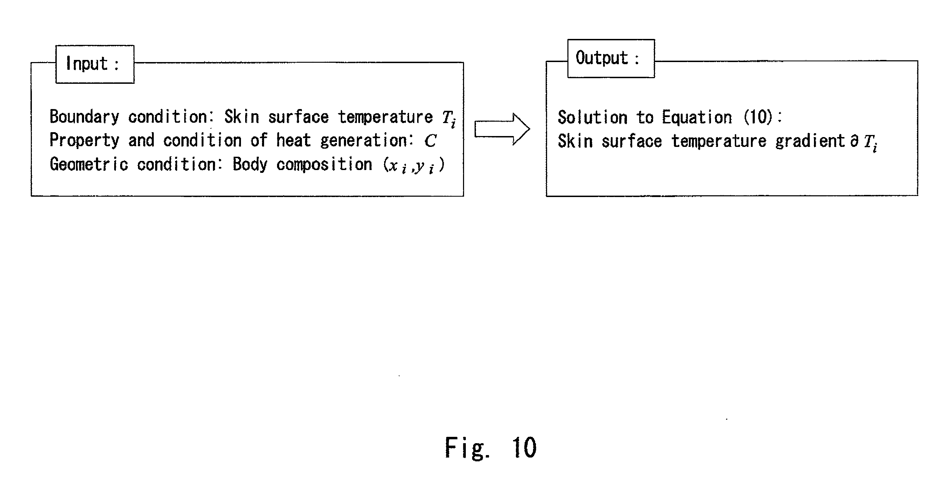

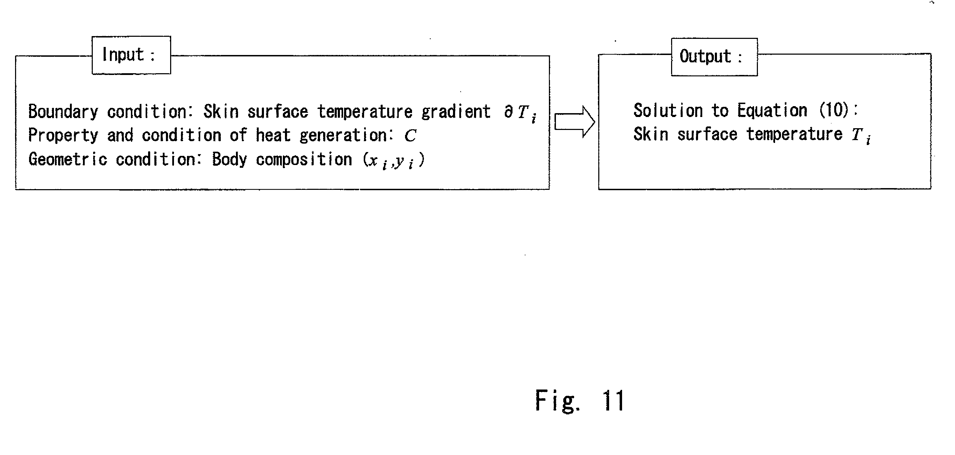

Flow of the information is drawn in Fig. 10 where input and output are separately written. Or when temperature gradients are given along the skin surface, flow of the information is expressed by Fig.11.

The above discussion is summarized as follows. There are four pieces of information involved in the heat conduction analysis, namely temperature $T_i$, temperature gradient $\partial T_i$, physical property-heat generation condition $C$ and body composition $(x_i, y_i)$. In the boundary element method, a relationship among the four pieces of information is derived from the governing equation, three of the four pieces of information are given as analysis conditions, and the remaining unknown piece of information is obtained.

(Use of inverse problem analysis based on the boundary element method, 1)

As, by utilising three known pieces of information, the remaining unknown piece of information can be obtained in the boundary element method, physical property-heat generation condition $C$ or body composition $(x_i, y_i)$ must also be obtained in turn.

Such analysis is called inverse problem analysis.

(In contrast, analysis in which temperature or temperature gradient is calculated as explained in (Outline of the boundary element method) is called forward problem analysis.)

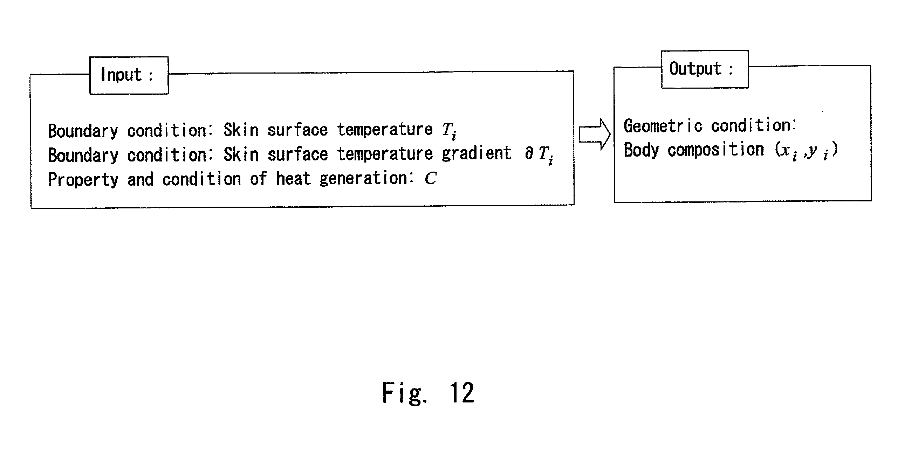

For example, when the body composition is treated as being unknown, flow of information can be understood as shown in Fig.12 where input and output are separately written. Simply put, the body composition can be calculated if distributions of temperature and temperature gradient along the skin surface are given at the same time as the boundary conditions.

Calculation of the body composition is certainly the purpose of the present invention. The process of evaluating the body composition can be put into practice with the inverse problem analysis.

The practical procedures based on the Newton-Rhaphson method are as follows.

In the beginning, the system of equation (12), in which temperatures are unknowns, is built with the given set of boundary temperature gradients imposed as the constraint. At this point, the given set of boundary temperatures is not under consideration.

The resultant solution obtained from Equation (12) is expressed as Equation (13).

$$T = A^{-1}f \quad \cdots\cdots \quad (13)$$

The solution shown in Equation (13) does not always agree with the given set of boundary temperatures $T^*$. On the other hand, one can have Equation (14) when differentiating Equation (12) with $x_i$.

$$\frac{\partial A}{\partial x_i} T + A \frac{\partial T}{\partial x_i} = \frac{\partial f}{\partial x_i} \quad \Rightarrow \quad \frac{\partial T}{\partial x_i} = A^{-1} \left( \frac{\partial f}{\partial x_i} - \frac{\partial A}{\partial x_i} T \right) \quad \cdots\cdots \quad (14)$$

Derivatives of boundary temperatures in terms of $x_i$ can be obtained in the form of the Jacobian matrix as shown in Equation (14).

Derivatives of boundary temperatures in terms of $x_i$ can be calculated in the same manner.

It is then assumed that solution $T$ in Equation (13) does not agree with the given set of boundary temperatures $T^*$ because of the wrong composition $(x_i, y_i)$. Thus, corrections to the compound $(\Delta x_i, \Delta y_i)$ are considered, and the corresponding change in the calculated boundary temperatures $\Delta T$ is estimated by Equation (15).

$$\Delta T = \sum_i \left( \frac{\partial T}{\partial x_i} \cdot \Delta x_i + \frac{\partial T}{\partial y_i} \cdot \Delta y_i \right) \quad \cdots\cdots \quad (15)$$

Let $\Delta T = T^* - T$ and the correct body compound can be calculated by solving Equation (15) for $(\Delta x_i, \Delta y_i)$.

Besides skin temperature, skin temperature gradient needs to be given beforehand so that the inverse problem analysis explained in this section is enabled. Evaluation of skin temperature gradient is fairly easy when skin temperature is already known. Heat transmitted towards the skin surface from the body core and that transferred from the skin surface to the surroundings are in balance. This thermal equilibrium is mathematically expressed by Equation (16).

$$-\lambda \cdot (\partial T)_s = h(T_s - T_{air}) \quad \cdots\cdots \quad (16)$$

Here, subscript $s$ represents the skin surface.

$\lambda$, $h$ and $T_{air}$ stand for thermal conductivity of the skin, heat transfer coefficient between the skin and the air, and temperature of the air, respectively.

The left hand side of Equation (16) represents heat flux due to heat conduction while the right hand side is heat flux removed from the skin surface to the surroundings.

Since skin temperature is known, temperature gradient $\partial T$ can be easily calculated from Equation (16).

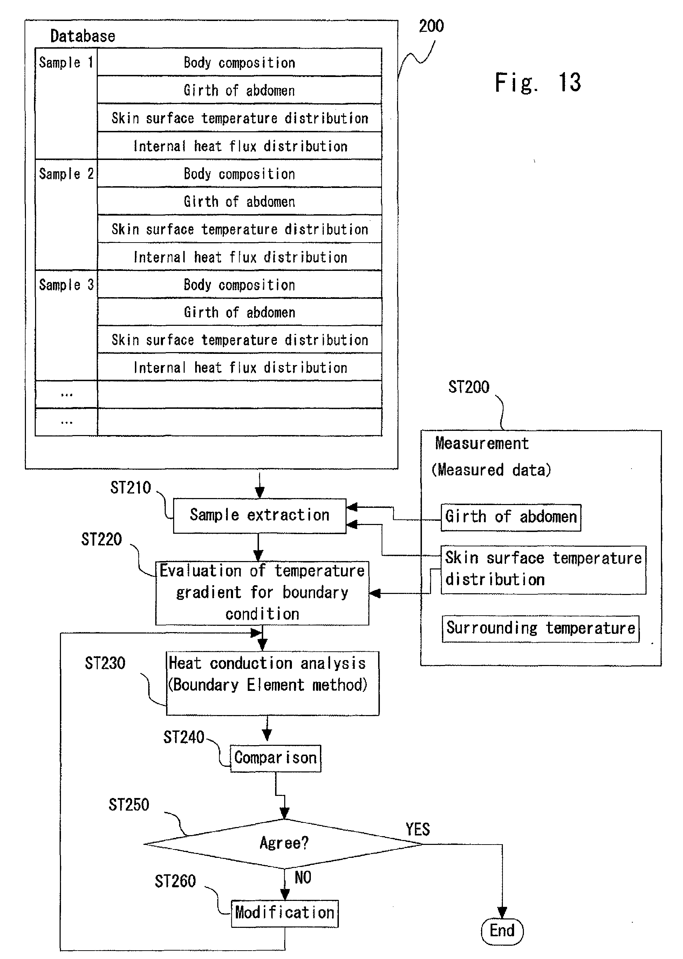

Fig.13 is a diagram showing the flow of process operations in the second exemplary embodiment.

In the same manner as in the first exemplary embodiment, it is necessary first of all to construct a database 200 for this exemplary embodiment.

Subsequently, abdomen girth and skin surface temperature of a patient whose body fat is going to be evaluated are measured, and the room temperature is also measured (ST200).

The skin surface temperature can be measured by thermography.

Subsequently, the sample closest to the patient in terms of abdomen girth and skin surface temperature is extracted from samples in the database (ST210).

As in the first exemplary embodiment, one possible approach in this process is to shortlist several promising samples with only abdomen girth, and then, to select one from the shortlisted samples referring to the averaged skin temperature. Furthermore, there is another approach in which shapes of skin temperature curves such as those shown in Fig.5 are examined: samples with skin temperature curves similar in shape to the patient's curve are chosen.

Subsequently, the skin temperature gradient of the patient is calculated from the measured skin temperature (ST220). This is realizable with Equation (16) as explained above.

Subsequently, heat conduction analysis is carried out on the body composition of the sample extracted in ST210 with the skin temperature gradient distribution imposed as the constraint, and a skin temperature distribution on the sample is calculated (ST230).

The distribution of skin temperature measured in ST200 and that calculated in ST 230 are compared (ST240).

When the two skin temperature distributions agree (ST250; Yes), the body composition used in the heat conduction analysis is considered to be the same as that of the patient, and the data processing is terminated.

If, on the other hand, the distribution of skin temperature calculated in ST230 disagrees with the distribution of actual skin temperature of the patient, the body composition is modified (ST260) and the skin temperature distribution on the sample is recalculated, with the data-processing returning to ST230. The processes from ST230 to ST260 are repeatedly carried out until the distributions of calculated and measured skin temperature agree with each other.

The correct body composition of the patient can be obtained through the procedures explained above.

Recall that, in the first exemplary embodiment 1, the data processing was terminated upon the body core temperature calculated in the sample agreeing with that of the patient. However, the body core temperature cannot always be measured directly and the exact position where it is defined is ambiguous. (If direct measurement of the body core temperature is conducted, the prospective position for conducting measurement will be the rectum, which means that the patient will feel extreme stress.)

However, in the second exemplary embodiment, termination of the data processing can be determined by comparing the calculated skin surface temperature with the real skin surface temperature of the patient, which latter temperature is more easily and accurately measureable than the former one. Thus, the second exemplary embodiment 2 enables more accurate evaluation of the body composition. It is another advantageous point as explained above that use of the boundary element method results in a much faster operation than the finite volume method.

(Modified Example 1)

(Use of inverse problem analysis based on the boundary element method, 2)

Exact values of thermal conductivity $\lambda$ of tissues in individual human bodies cannot be acquired with ease. 0.4 W/ (m·K) for muscle and skin and 0.2W/(m·K) for fat are assumed in the above discussion because these approximate values have been widely used. However, they are not exact values.

A method of determination of internal heat generation $q_{in}$ is provided in the first exemplary embodiment. Although there are good physical grounds for this method, there is still room for doubt about whether variation in $q_{in}$ due to age, gender and physique can be taken into account precisely.

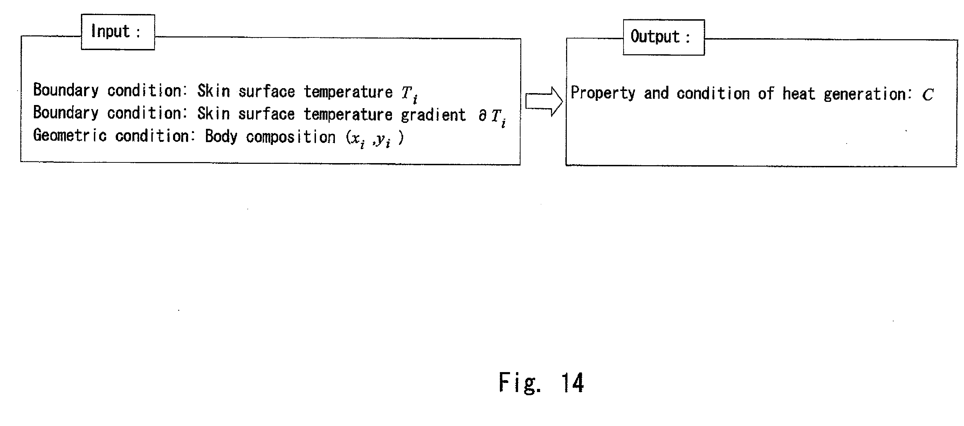

Thus, more realistic values of $C = q_{in}/\lambda$ should be calculated by carrying out inverse problem analysis shown in Fig.14 upon construction of the database.

The three pieces of input information including the exact body composition are available because skin surface temperature measurement and CT scanning are conducted for construction of the database 200. (In this regard, since numerical procedures for inverse problem analysis were discussed above, no further explanation is given here.)

Values of $C$ collected in this manner are classified according to age, gender, height and weight before being stored in the database, and should be utilized in the heat conduction analysis (ST230) shown in Fig.13.

As discussed above, exact physical properties of each portion, which remained unknown before, can be obtained by conducting inverse problem analysis based on the boundary element method on tissues of the human body.

(Modified Example 2)

(Measurement of body core temperature and its utilization)

Body core temperature is used for reference in the first exemplary embodiment.

Body core temperature is usually regarded as being the same as rectal temperature. However, most patients will find measurement of rectal temperature so stressful that this is considered an unfavorable approach. As the rectum is located anatomically next to the bladder, it can be assumed that there is no difference in temperature between the two organs. Therefore, urinary temperature can be used as a substitute for body core temperature.

As long as the procedures in the second exemplary embodiment are followed, it is not necessary to refer to body core temperature in calculating body composition. However, information about body core temperature is useful for compensation of the error arising in measurement of skin surface temperature. For example, the condition that the average temperature of the intestines is equal to the body core temperature is imposed in inverse problem analysis as a constraint.

The present invention is not limited to the exemplary embodiments described above, and various changes and modification may be made without departing from the scope of the invention.

Physical values used in the heat conduction analysis such as internal heat generation, body core temperature and thermal conductivities of fat and skin should be properly determined by referring to literature or experimental data. Note that the values cited above in this document are only examples.

In regard of the above exemplary embodiments, the invention was mainly explained in terms of methodology. However, an apparatus can also be made by integrating a storage device for the database and a computer for data processing.

In other words, it is possible to use a computer, on which a prearranged program is installed, as a means for sample extraction, a numerical heat conduction analysis, comparison and modification of the body composition.

**Claims**

1.  A method of body fat measurement comprising:

    a measuring step of measuring a distribution of skin surface temperature of a subject;
    a first operation step evaluating temperature gradient on the skin surface based on the measured skin temperature;
    a second operation step calculating a skin temperature distribution by conducting numerical heat conduction analysis on a prearranged sample body composition with the temperature gradient imposed as a boundary condition;
    a comparing step comparing the skin temperature distributions obtained in the measuring and second operation steps;
    a modification step modifying the body composition of the sample in a case where the two skin temperature distributions disagree in the comparing step; and
    repeating the second operation step and the modification step to make the two skin temperature distributions sufficiently close.

2.  The method of body fat measurement according to Claim 1, further comprising:

    prearranging database in which abdomen girth , body composition and skin surface temperature of each sample is recorded; and
    extracting a sample closest to the measured subject from the database by referring to the real abdomen girth and skin surface temperature of the subject obtained in the measuring step.

3.  The method of body fat measurement according to Claim 1, further comprising:

    measuring body surface temperature and body composition of real human bodies,
    calculating skin surface gradient based on the measured surface temperature and the measured body composition of real human bodies,
    evaluating thermal properties of respective tissues based on the measured surface temperature, the measured body composition and the calculated skin surface gradient; and
    constructing a database that classifies the thermal properties of respective tissues according to a single or multiple attributes of the sample chosen based on age, gender, height and weight.

4.  The method of body fat measurement according to Claim 1, wherein the boundary condition further includes a condition that average temperature of intestines agrees with measured body core temperature.

5.  An apparatus for body fat measurement comprising:

    a measuring means that measures a distribution of skin surface temperature of a subject;
    a first operation means that evaluates temperature gradient on the skin surface based on the measured skin temperature;
    a second operation means that calculates skin temperature distribution by conducting a numerical heat conduction analysis on a prearranged sample body composition with the temperature gradient calculated by the first operation means imposed as a boundary condition;
    a comparing means that compares the measured and calculated skin temperature distributions; and
    a modifying means that modifies the body composition of the sample in a case where a difference between the two skin temperature distributions is detected by the comparing means; wherein, until the calculated skin temperature distribution is sufficiently close to the measured skin temperature distribution, the modification to the sample body composition by the modification means and the calculation of the skin surface temperature distribution by the second operation means are repeated.

**Patentansprüche**

1. Verfahren zur Körperfettmessung, das Folgendes umfasst:

   einen Messschritt zum Messen einer Verteilung der Hautoberflächentemperatur einer Person;
   einen ersten Verfahrensschritt zum Auswerten des Temperaturgradienten auf der Hautoberfläche basierend auf der gemessenen Hauttemperatur;
   einen zweiten Verfahrensschritt zum Berechnen einer Hauttemperaturverteilung, indem eine numerische Wärmeleitanalyse an einer vorbereiteten Körperzusammensetzungsprobe durchgeführt wird, wobei der Temperaturgradient als Randbedingung verwendet wird;
   einen Vergleichsschritt zum Vergleichen der Hauttemperaturverteilungen, die bei dem Messschritt und dem zweiten Verfahrensschritt erhalten wurden;
   einen Modifikationsschritt zum Modifizieren der Körperzusammensetzung der Probe in einem Fall, in dem die beiden Hauttemperaturverteilungen bei dem Vergleichsschritt nicht übereinstimmen; und
   Wiederholen des zweiten Verfahrensschritts und des Modifikationsschritts, um die beiden Hauttemperaturverteilungen ausreichend aneinander anzunähern.

2. Verfahren zur Körperfettmessung nach Anspruch 1, das ferner Folgendes umfasst:

   Vorbereiten einer Datenbank, in der Bauchumfang, Körperzusammensetzung und Hautoberflächentemperatur jeder Probe aufgezeichnet werden; und
   Extrahieren einer Probe, die der gemessenen Person am meisten entspricht, aus der Datenbank, indem auf den realen Bauchumfang und die Hautoberflächentemperatur der Person, der bzw. die in dem Messschritt erhalten wurden, Bezug genommen wird.

3. Verfahren zur Körperfettmessung nach Anspruch 1, das ferner Folgendes umfasst:

   Messen der Körperoberflächentemperatur und der Körperzusammensetzung von realen menschlichen Körpern,
   Berechnen des Hautoberflächengradienten basierend auf der gemessenen Oberflächentemperatur und der gemessenen Körperzusammensetzung von realen menschlichen Körpern,
   Bewerten der thermischen Eigenschaften der jeweiligen Gewebe basierend auf der gemessenen Oberflächentemperatur, der gemessenen Körperzusammensetzung und des berechneten Hautoberflächengradienten; und
   Erstellen einer Datenbank, die die thermischen Eigenschaften der jeweiligen Gewebe gemäß einem einzelnen Attribut oder mehrerer Attribute der gewählten Probe klassifiziert, basierend auf Alter, Geschlecht, Körpergröße und Gewicht.

4. Verfahren zur Körperfettmessung nach Anspruch 1, wobei die Randbedingung ferner eine Bedingung umfasst, dass die Durchschnittstemperatur des Darms mit der gemessenen Körperkerntemperatur übereinstimmt.

5. Vorrichtung zur Körperfettmessung, die Folgendes umfasst:

   ein Messmittel zum Messen einer Verteilung der Hautoberflächentemperatur einer Person;
   ein erstes Verfahrensmittel zum Auswerten des Temperaturgradienten auf der Hautoberfläche basierend auf der gemessenen Hauttemperatur;
   ein zweites Verfahrensmittel zum Berechnen einer Hauttemperaturverteilung, indem eine numerische Wärmeleitanalyse an einer vorbereiteten Körperzusammensetzungsprobe durchgeführt wird, wobei der Temperaturgradient, der durch die erste Verfahrensvorrichtung berechnet wird, als Randbedingung verwendet wird;
   ein Vergleichsmittel, das die gemessene und die berechnete Hauttemperaturverteilung vergleicht; und
   ein Modifizierungsmittel, das die Körperzusammensetzung der Probe in einem Fall modifiziert, in dem das Vergleichsmittel einen Unterschied zwischen den beiden Hauttemperaturverteilungen erfasst; wobei die Modifikation der Körperzusammensetzungsprobe durch das Modifizierungsmittel und die Berechnung der Hautoberflächentemperaturverteilung durch das zweite Verfahrensmittel wiederholt werden, bis die berechnete Hauttemperaturverteilung ausreichend nahe an der gemessenen Hauttemperaturverteilung liegt.

**Revendications**

1. Procédé de mesure de la graisse corporelle, comprenant :

une étape de mesure consistant à mesurer une distribution de la température de surface épidermique chez un sujet ;

une première étape d'exploitation évaluant le gradient de température sur la surface épidermique, sur la base de la température épidermique ayant été mesurée ;

une deuxième étape d'exploitation calculant une distribution de la température épidermique grâce à la réalisation d'une analyse numérique de la conduction de chaleur sur une composition corporelle échantillon agencée au préalable, alors que le gradient de température est imposé à titre de condition limite ;

une étape de comparaison comparant les distributions de température épidermique qui ont été obtenues lors des étapes de mesure et de deuxième exploitation ;

une étape de modification modifiant la composition corporelle de l'échantillon dans un cas où les deux distributions de la température épidermique ne concordent pas lors de l'étape de comparaison ; et

répéter la deuxième étape d'exploitation et l'étape de modification afin de rendre les deux distributions de la température épidermique suffisamment proches.

2. Procédé de mesure de la graisse corporelle selon la revendication 1, comprenant en outre les opérations consistant à :

agencer au préalable une base de données dans laquelle sont enregistrés le tour d'abdomen, la composition corporelle et la température de surface épidermique de chaque échantillon ; et

extraire à partir de la base de données un échantillon qui se rapproche le plus du sujet mesuré, en se reportant au tour d'abdomen et à la température de surface épidermique réels du sujet obtenus lors de l'étape de mesure.

3. Procédé de mesure de la graisse corporelle selon la revendication 1, comprenant en outre les opérations consistant à :

mesurer la température de surface corporelle et la composition corporelle de corps humains réels,

calculer un gradient de surface épidermique sur la base de la température de surface mesurée et de la composition corporelle mesurée chez des corps humains réels,

évaluer des propriétés thermiques de tissus respectifs, sur la base de la température de surface mesurée, de la composition corporelle mesurée et du gradient de surface épidermique calculé ; et

élaborer une base de données qui classifie les propriétés thermiques de tissus respectifs, en conformité avec un attribut unique ou de multiples attributs de l'échantillon choisi sur la base de l'âge, du sexe, de la taille et du poids.

4. Procédé de mesure de la graisse corporelle selon la revendication 1, la condition limite incluant en outre une condition suivant laquelle une température moyenne des intestins concorde avec la température profonde du corps ayant été mesurée.

5. Appareil destiné à mesurer la graisse corporelle, comprenant :

un moyen de mesure qui mesure une distribution de la température de surface épidermique chez un sujet ;

un premier moyen d'exploitation qui évalue le gradient de température sur la surface épidermique, sur la base de la température épidermique ayant été mesurée ;

un deuxième moyen d'exploitation qui calcule une distribution de la température épidermique grâce à la réalisation d'une analyse numérique de la conduction de la chaleur sur une composition corporelle échantillon agencée au préalable, alors que le gradient de température calculé par le premier moyen d'exploitation est imposé à titre de condition limite ;

un moyen de comparaison qui compare les distributions de température épidermique ayant été mesurées et calculées ; et

un moyen de modification qui modifie la composition corporelle de l'échantillon dans un cas où le moyen de comparaison détecte une différence entre les deux distributions de la température épidermique ; cas dans lequel la modification apportée à la composition corporelle échantillon par le moyen de modification et le calcul de la distribution de la température épidermique par le deuxième moyen d'exploitation sont répétés jusqu'à ce que la distribution de la température épidermique calculée soit suffisamment proche de la distribution de la température épidermique mesurée.

Fig. 1A

Fig. 1B

## Fig. 2A

## Fig. 2B

Fig. 3A

FRONT

BACK

Fig. 3B

FRONT

BACK

Fig. 4A

27.5  29.0  30.5  32.0  33.5  35.0  36.5

Fig. 4B

27.5  29.0  30.5  32.0  33.5  35.0  36.5

Fig. 5

Fig. 6A

Fig. 6B

Fig. 7

| Database | | |
|---|---|---|
| Sample 1 | Body composition | |
| | Girth of abdomen | |
| | Skin surface temperature distribution | |
| | Internal heat flux distribution | |
| Sample 2 | Body composition | |
| | Girth of abdomen | |
| | Skin surface temperature distribution | |
| | Internal heat flux distribution | |
| Sample 3 | Body composition | |
| | Girth of abdomen | |
| | Skin surface temperature distribution | |
| | Internal heat flux distribution | |
| ... | | |
| ... | | |

200

Patient

ST100 — Measurement
(Measured data)

Girth of abdomen

Skin surface temperature distribution

Body core temperature

ST110 — Sample extraction

ST120 — Heat conduction analysis

ST130 — Comparison

ST140 — Agree?
  YES
  NO

ST150 — Modification

End

Fig. 8

Fig. 9

Input :

Boundary condition: Skin surface temperature $T_i$
Property and condition of heat generation: $C$
Geometric condition: Body composition $(x_i, y_i)$

$\Rightarrow$

Output :

Solution to Equation (10):
Skin surface temperature gradient $\partial T_i$

Fig. 10

Input :

Boundary condition: Skin surface temperature gradient $\partial T_i$
Property and condition of heat generation: $C$
Geometric condition: Body composition $(x_i, y_i)$

$\Rightarrow$

Output :

Solution to Equation (10):
Skin surface temperature $T_i$

Fig. 11

Input :

Boundary condition: Skin surface temperature $T_i$
Boundary condition: Skin surface temperature gradient $\partial T_i$
Property and condition of heat generation: $C$

Output :

Geometric condition:
Body composition $(x_i, y_i)$

Fig. 12

Fig. 13

| Database | | |
|---|---|---|
| Sample 1 | Body composition | |
| | Girth of abdomen | |
| | Skin surface temperature distribution | |
| | Internal heat flux distribution | |
| Sample 2 | Body composition | |
| | Girth of abdomen | |
| | Skin surface temperature distribution | |
| | Internal heat flux distribution | |
| Sample 3 | Body composition | |
| | Girth of abdomen | |
| | Skin surface temperature distribution | |
| | Internal heat flux distribution | |
| ... | | |
| ... | | |

200

ST200

Measurement
(Measured data)

Girth of abdomen

Skin surface temperature
distribution

Surrounding temperature

ST210 — Sample extraction

ST220 — Evaluation of temperature gradient for boundary condition

ST230 — Heat conduction analysis (Boundary Element method)

ST240 — Comparison

ST250 — Agree?   YES

ST260 — Modification   NO

End

29

Input :

Boundary condition: Skin surface temperature $T_i$
Boundary condition: Skin surface temperature gradient $\partial T_i$
Geometric condition: Body composition $(x_i, y_i)$

Output :

Property and condition of heat generation: $C$

Fig. 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005152061 A
- JP 2011025071 A
- JP 2009261435 A

**Non-patent literature cited in the description**

- **J.A. HARRIS ; F.G. BENEDICT.** A Biometric Study of Basal Metabolism in Man. The Carnegie Institution, 1979
- **S. YOKOYAMA.** Heat Transfer Phenomena in Living bodies. Hokkaido University Press, 1993
- **C.D. MURRAY.** The Physiological Principle of Minimum Work: I. The Vascular System and the Cost of Blood Volume. *Proceedings of the National Academy of Sciences of the United States of America,* 1926, vol. 12 (3), 207-214